# EUROPEAN PATENT SPECIFICATION

(11) **EP 1 519 769 B1**
(45) Date of publication and mention of the grant of the patent: **07.02.2007**
(21) Application number: 03732753.3
(22) Date of filing: 25.06.2003
(51) Int. Cl.: A61M 27/00

(54) **WOUND FLUID COLLECTING AND INDICATING DEVICE**
VORRICHTUNG ZUM ABFÜHREN VON WUNDSEKRETEN KOMBINIERT MIT EINER ANZEIGEVORRICHTUNG
DISPOSITIF COLLECTEUR ET INDICATEUR D'EXSUDAT DE PLAIE

(30) Priority: 25.06.2002 GB 0214665
(43) Date of publication of application: 06.04.2005
(73) Proprietor: Johnson & Johnson Medical Ltd., Edinburgh EH2 4NH (GB)
(72) Inventor: Watt, Paul William, West Yorkshire BD20 6UN (GB); Clark, Rachael Louise, North Yorkshire BD23 2DH (GB)
(74) Representative: James, Anthony Christopher W.P.
(86) International application number: PCT/GB2003/002725
(87) International publication number: WO 2004/000409

(56) References cited:
- WO-A-01/16576
- WO-A-91/08793
- US-A- 4 553 966
- US-A1- 2002 016 577

## Description

The present invention relates to an apparatus for collecting and mixing wound fluid from a plurality of locations in a wound, and for detecting the presence of a wound fluid marker in the collected wound fluid.

An apparatus comprising the features specified in the preamble of claim 1 is disclosed in US-A-2002/0016577.

Monitoring wound healing conditions or progress using factors such as enzymes, growth factors, or bacterial factors, may prove to be a simple and effective way to determine whether a wound is infected, about to be infected, healing, static or regressing. This would provide important clinical benefits.

It has been found that the concentration of wound factors such as certain proteins and glycosaminoglycans vary considerably over the area of a single chronic wound such as a venous ulcer, pressure sore or diabetic ulcer. This makes monitoring wound progress by direct sampling or analysis at single sites within the wound unreliable and misleading. In order to analyse a representative sample of chronic wound fluid it is possible to drain off the fluid for mixing and analysis at a remote location, but this is impractical, especially for wounds producing relatively little exudate such as chronic wounds.

The present invention provides an apparatus comprising: a distributed inlet for collecting and mixing wound fluid from a plurality of locations in a wound; and a sensing or indicating device for detecting the presence of a wound fluid marker in the wound fluid mixture.

By collecting wound fluid from a plurality of locations in the wound, and preferably from substantially the whole surface area of the wound, and analysing for the wound factor *in situ,* the invention enables a quick, global assessment of the wound state to be made at low cost and even for low to medium exuding wounds.

The distributed inlet preferably comprises a plurality of spaced apart inlet tubes, pores or capillaries joined to a manifold for combining and mixing the collected wound fluid. In certain embodiments the distributed inlet is a collection of capillary tubes held in spaced-apart fashion in a housing, the plurality of tubes leading to a manifold that mixes and guides the liquid to the indicator device by capillary action, or under the influence of suction. In other embodiments the distributed inlet may comprise an absorbent material that allows liquid to be collected over an extended area. The absorbent material must be chosen so that it does not adsorb any of the wound markers of interest. In these embodiments the apparatus usually comprises a suction device, such as a syringe or a pump, to draw the absorbed fluid out of the absorbent and into the indicator device.

Mixing of the wound fluid from the plurality of locations preferably takes place in a mixing channel or chamber upstream of the indicator device, but in some embodiments it may take place at the indicator device.

Preferably, the distributed inlet is suitable for collecting wound fluid from a plurality of locations located at least 1cm apart in a wound, preferably at least 2cm apart. Preferably, each one of the plurality of inlet tubes/pores/capillaries is spaced no more than about 1cm from the next adjacent inlet tube at the surface of the apparatus, preferably no more than about 5mm from the next adjacent inlet tube. Preferably, there are at least 4 inlets at the surface of the apparatus for receiving wound fluid, and more preferably there are at least about 10 such inlets. Preferably, the inlet is adapted to take up wound fluid from an area of at least 4cm², more preferably at least 10cm².

In certain embodiments the distributed inlet may be a conformable body for insertion into a cavity wound or a sinus. In certain embodiments the distributed inlet comprises a sheet having a plurality of apertures therein for application to a topical wound, such as a chronic ulcer. The sheet may be conformable to cover the wound and the surrounding skin. The total area of the distributed inlet in the sheet form is preferably at least 10cm², more preferably at least 25cm², and most preferably at least 50cm². In certain embodiments, the distributed inlet is formed from relatively non-liquid-absorbent material, whereby the wound fluid flows into the apertures and to the analysis device without being substantially absorbed by the inlet material. In other embodiments, the distributed inlet may comprise liquid-absorbent material, but these embodiments preferably also comprise a suction device to draw the wound fluid out of the absorbent material and into the detector. The inlet may comprise a substantially non-absorbent cushioning material, such as a closed-cell foam.
In certain embodiments the distributed inlet comprises a capillary collector device. That is to say, the apertures and inlet manifold are of sufficiently small diameter that wound fluid is withdrawn from the wound surface and mixed and drawn up to the analysis device by capillary action. Alternatively, or additionally, the apparatus according to the present invention may comprise a device for applying suction to the distributed inlet to draw wound fluid into the inlet and over the analysis device. Such suction may be applied by a hand bulb, or by conventional wound drainage suction apparatus, or by applying a higher vacuum to the wound (which also assists wound healing), for example as described in EP-A-0620720.

The use of a large-area collector enables an averaged mixture of wound fluid to be obtained from relatively low or medium exuding wounds. In some cases it may even so be desirable to increase the volume of liquid being passed to the sensing or indicating device, and this can be done by supplying a washing liquid, such as a sterile saline, to the wound surface in a distributed fashion in order to dilute and wash out the wound fluid. In these embodiments the apparatus may further comprises a liquid supply port for supplying such a liquid to the wound. Preferably, the liquid supply port is linked to a distributed outlet on the wound facing side of the apparatus through a suitable manifold, and some embodiments the distributed outlet may be the same as the distributed inlet by use of a suitable valve arrangement to alternate the supply and removal of liquid from the wound surface.

The apparatus according to the present invention further comprises a sensing or indicating device for detecting the presence of a wound fluid marker in the wound fluid mixture. The device is integral to the apparatus according to the invention, and therefore removes the need for wound fluid analysis at a remote location. Preferably, the device undergoes a visible change (which term includes a change that is visible under ultra-violet light) in the presence of the wound fluid marker. Accordingly, the sensing or indicating device is preferably visible through a transparent side wall of the apparatus so as to give a visible indication to a patient or care giver.

Preferably, the sensing or indicating device contains one or more immunological binding partners to bind one or more indicator molecules present in the wound fluid. The immunological binding partners may for example comprise monoclonal or polyclonal antibodies, antibody fragments, or chimeric antibodies. Alternatively, the immunological binding partners may comprise antigens in cases where the presence of predetermined antibodies in the wound fluid is being detected. Preferably, the immunological binding partners comprise monoclonal antibodies. Preferably, the immunological other binding partners are immobilised on a support, for example by avidin-biotin linking or dialdehyde derivatisation of the support material followed by cross-linking to a peptide binding partner.

The support comprising immunological or other binding partners may be used in a range of immunoassays to detect the presence of biologically active molecules in the wound fluid. For example, the supports having antibodies or antibody fragments bound thereto may be used in sandwich immunoassay-type detection.

Alternatively, the substrate may have analog ligands bound to the antibodies, whereby the molecules present in the wound fluid are detected by affinity displacement immunoassay. Various other immunoassays will be apparent to persons skilled in the art.

In other preferred embodiments, the sensing or detecting device in the apparatus according to the present invention comprises a chemiluminescent chromogenic or fluorogenic substrate for an enzyme present in the wound fluid.

The sensing or indicating device in the apparatus according to the present invention can be used to detect a wide variety of molecules that are present in wound fluid. The device may also measure pH and/or dissolved gases.

Preferably, the device detects one or more molecules that are indicative of a wound healing disorder or infection. For example, the device may detect one or more molecules selected from the group consisting of: protease enzymes, collagen propeptides, collagen telopeptides and collagen crosslinks such as pyrridinoline, protease inhibitors, plasmin, lactate dehydrogenase, cathepsins, cytokins, peroxidase enzymes, cortisol free radicals, and growth factors. Preferably, the device detects one or more protease enzymes such as matrix metalloproteinases, low molecular weight gelatinase and latent or active elastases. For example, the device could detect matrix metalloproteinase 2 (MMP2) or matrix metalloproteinase 9 (MMP9). The protease enzymes are preferably detected by reaction with chromogenic or fluorogenic substrates bound to the detector device, such as Dnp-Pro-β-cyclohexyl-Ala-Gly-Cys(Mu)-His-Ala-Lys-(N-Me-Abz)-NH₂ (available from Bachem Inc., for MMP9 and 1). Other chromogenic substrates such as gelatin-Texas Red and gelatin-Azo Brilliant Blue could be used to detect these molecules.

In other embodiments, the device detects an alkaline phosphatase (ALP) enzyme present in the wound. It is known that extracellular ALP levels are elevated in healing wounds. The device could, for example, comprise a substrate for ALP such as p-nitrophenyl phosphate. Alternatively or additionally, the detector could comprise an immunological binding partner for ALP.

Another preferred apparatus according to the present invention detects the presence of elevated levels of elastase in the wound fluid, since this has been found to be a good indicator of pre-clinical wound infection.

In a further aspect the present invention provides a method of determining the condition of a wound comprising applying an apparatus according to the present invention to a surface of the wound, and using the apparatus to detect the presence or absence of a wound marker in a wound fluid mixture received by the apparatus. Preferably, the apparatus is applied to the surface of the wound for a period of from 1 minute to 1 day, more preferably from 10 minutes to 1 hour. This may be dictated by levels of exudates for purpose of maximal utility.

In those embodiments in which suction is used, a dressing is usually applied over the apparatus, and in certain preferred embodiments the dressing is essentially airtight, and suction is applied through the apparatus to lower the pressure at the wound surface to a level below about 700mm Hg absolute, preferably below about 680mm Hg absolute. In certain embodiments, suction may be applied through the apparatus in order to draw wound fluid into the apparatus and over the detector.

An embodiment of the present invention will now be described further, by way of example, with reference to the accompanying drawings, in which:-
Figure 1 shows a schematic longitudinal cross-section through a first apparatus according to the present invention for application to a large-area wound.
Figure 2 shows a schematic longitudinal cross-section through a second apparatus according to the present invention having a suction device to draw wound fluid through the apparatus;
Figure 3 shows a schematic longitudinal cross-section through a third apparatus according to the present invention that has been applied to a sinus cavity wound; and
Figure 4 shows a schematic longitudinal cross-section through a fourth apparatus according to the present invention that has been applied to a cavity wound.

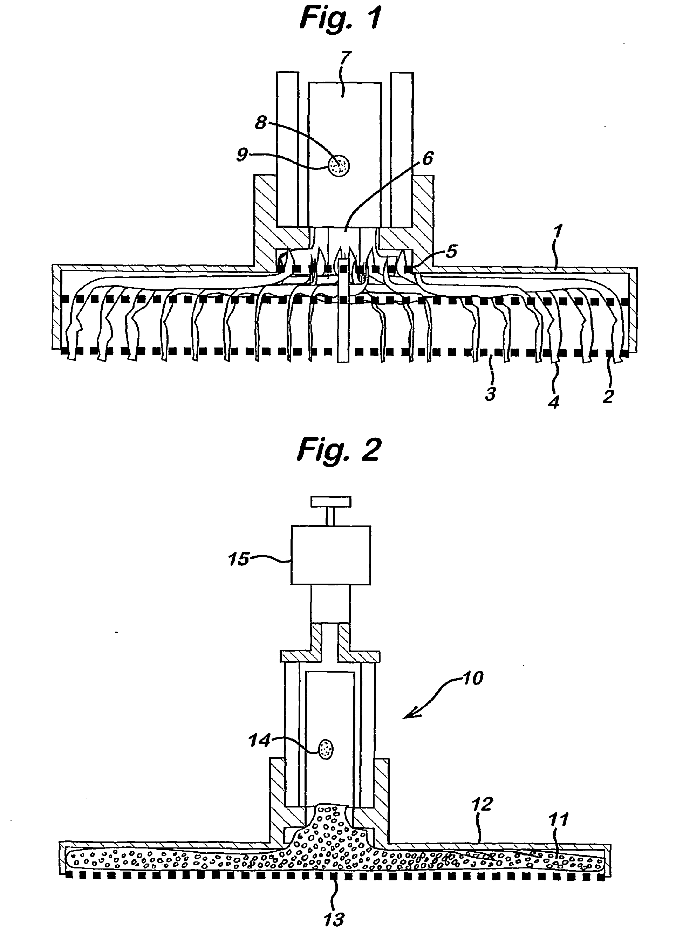

Referring to Figure 1, the apparatus comprises a semi-rigid disk housing 1 of polyethylene having a diameter of approximately 5cm and a thickness of approximately 10mm. A lower surface 2 has an array of openings 3 each holding the open end of a respective capillary tube 4 made from flexible polypropylene of 1 mm diameter. The capillary tubes 4 are gathered together into a manifold 5 leading to a mixing duct 6. The mixing duct 6 leads to a compartment 7 containing an indicator strip 8, and having a transparent window 9.

In use, the apparatus is applied to a wound bed, and wound fluid is drawn out of the wound bed through the capillaries and mixing duct to the indicator strip.

The indicator strip is formed by soaking a sheet of Whatman No. 1 filter paper overnight at room temperature in a 1 millimolar aqueous solution of the elastase chromogenic substrate MeO-Suc-Ala-Ala-Pro-Val-(p-nitroanilide) supplied by Calbiochem Limited. This substrate releases yellow coloured p-nitroanilide dye when the peptide sequence is cleaved by an elastase enzyme. The filter paper is dried at 40°C following the soaking step.

Referring to Figure 2, the apparatus 10 shown consists of an absorbent or wicking sponge material 11 such as polyurethane foam encased in a polyethylene molded disc housing 12. The face 13 of the disc housing that will contact the wound fluid is permeable (perforated) to allow fluid to soak into the absorbent 11. The wound fluid is drawn out of the absorbent foam, through a mixing channel, and over the indicator device 14 by use of a syringe 15.

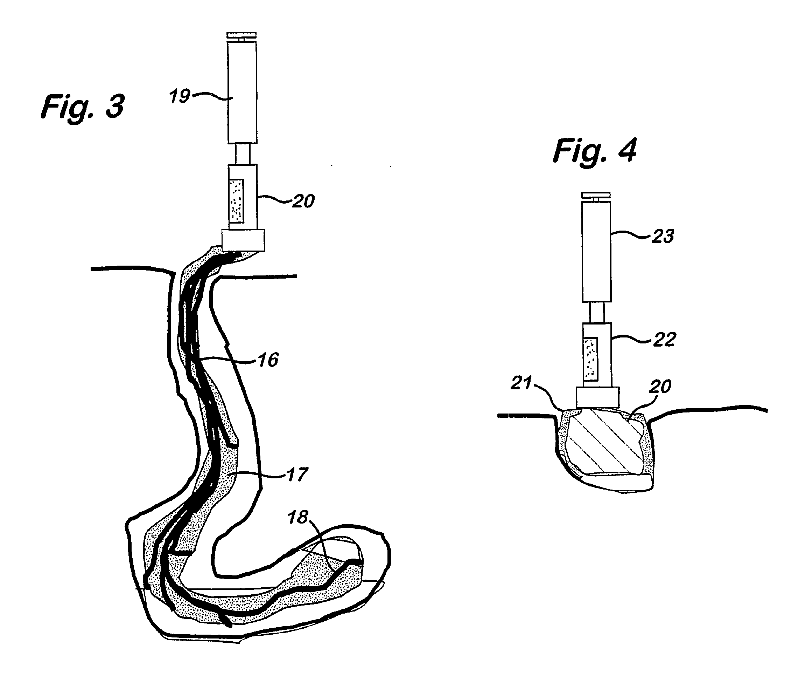

Referring to Fig 3, a plurality of capillary tubes 16 similar to those in the apparatus of Example 1 extend within a spunbonded nylon pouch 17 to different lengths in order to ensure adequate collection of fluid throughout a sinus. The greatest concentration of capillary tubes are at the end 18 of the flexible device where wound fluid is most likely to pool. Fluid collection is assisted by use of a syringe 19 attached to the pouch. This action will also draw the fluid over the indicator device 20.

Referring to Fig. 4, an absorbent material 20 such as polyurethane sponge is contained within a spunbonded nylon pouch 21 connected to a device containing an indicator strip 22 and further attached to a syringe 23 to draw the wound fluid over the indicator strip.

The above embodiments have been described by way of example. Many other embodiments falling within the scope of the accompanying claims will be apparent to the skilled reader.

## Claims

1. An apparatus comprising:
a distributed inlet (3, 6) for collecting and mixing wound fluids from a plurality of locations in a wound to provide a wound fluid mixture; **characterized by**
a sensing or indicating device (8) in fluid communication with said distributed inlet (3,6) for detecting the presence of a wound fluid marker in the wound fluid mixture.

2. An apparatus according to claim 1, wherein the distributed inlet is selected from the group consisting of a plurality of spaced apart capillary tubes, or a body of absorbent material, or a combination thereof.

3. An apparatus according to claim 1 or 2, wherein the distributed inlet is in the form of a sheet or plate having an area of at least 10cm².

4. An apparatus according to any preceding claim, further comprising a device for applying suction to the distributed inlet.

5. An apparatus according to any preceding claim, further comprising a liquid supply port to supply a washing liquid to the wound.

6. An apparatus according to any preceding claim, wherein the sensing or indicating device is visible through a side wall of the apparatus, and undergoes a visible change in the presence of said wound fluid marker.

7. An apparatus according to any preceding claim wherein the sensing or indicating device contains one or more immunological binding partners to bind one or more marker molecules present in the wound fluid.

8. An apparatus according to any preceding claim wherein the sensing or indicating device comprises a chemiluminescent chromogenic or fluorogenic substrate for an enzyme present in the wound fluid.

9. An apparatus according to any preceding claim wherein the sensing or indicating device detects one or more molecules selected from the group consisting of: protease enzymes, collagen propeptides, collagen telopeptides and collagen crosslinks such as pyrridinoline, protease inhibitors, plasmin, lactate dehydrogenase, cathepsins, cytokins, peroxidase enzymes, cortisol free radicals, and growth factors.

10. An apparatus according to any preceding claim wherein the sensing or indicating device detects one or more enzymes selected from the group consisting of matrix metalloproteinases, low molecular weight gelatinase, latent or active elastases and alkaline phosphatase (ALP).

## Patentansprüche

1. Vorrichtung mit:
einer verteilten Einlaßöffnung (3, 6) zum Abführen und Mischen von Wundsekreten von mehreren Bereichen in einer Wunde, um eine Wundsekretmischung bereitzustellen;
einer Abtast- oder Anzeigevorrichtung (8), welche mit der verteilten Einlaßöffnung in Fluß-Verbindung steht, zum Detektieren der Anwesenheit eines Wundsekretmarkers in der Wundsekretmischung.

2. Vorrichtung nach Anspruch 1, **dadurch gekennzeichnet, daß** die dezentralisierte Einlaßöffnung aus mehreren voneinander getrennten Kapillarrohren gebildet ist oder einem Körper eines absorbierenden Materials oder einer Kombination hiervon.

3. Vorrichtung nach Anspruch 1 oder 2, **dadurch gekennzeichnet, daß** die dezentralisierte Einlaßöffnung die Form eines Blattes oder einer Platte mit einer Fläche von mindestens 10 cm² aufweist.

4. Vorrichtung nach einem der vorangehenden Ansprüche, **gekennzeichnet durch** eine Saugvorrichtung an der dezentralisierten Einlaßöffnung.

5. Vorrichtung nach einem der vorangehenden Ansprüche, **gekennzeichnet durch** einen Flüssigkeitsbereitstellungsanschluß zum Bereitstellen einer Waschflüssigkeit für die Wunde.

6. Vorrichtung nach einem der vorangehenden Ansprüche, **dadurch gekennzeichnet, daß** die Abtast- oder Anzeigevorrichtung durch eine Seitenwand sichtbar ist und eine sichtbare Änderung durchläuft in Anwesenheit des Wundsekretmarkers.

7. Vorrichtung nach einem der vorangehenden Ansprüche, **dadurch gekennzeichnet, daß** die Abtast- oder Anzeigevorrichtung einen oder mehrere immunologisch bindende Partner aufweist, um ein oder mehrere in dem Wundsekret anwesende Markermoleküle zu binden.

8. Vorrichtung nach einem der vorangehenden Ansprüche, **dadurch gekennzeichnet, daß** die Abtast- oder Anzeigevorrichtung ein chemilumineszentes chromogenes oder fluorogenes Substrat für ein in dem Wundsekret anwesendes Enzym aufweist.

9. Vorrichtung nach einem der vorangehenden Ansprüche, **dadurch gekennzeichnet, daß** die Abtast- oder Anzeigevorrichtung ein oder mehrere Moleküle ausgewählt aus der folgenden Gruppe von Molekülen detektiert: Protease Enzyme, Kollagenpropeptide, Kollagentelopeptide und Kollagenverbindungen, wie zum Beispiel Pyrridinoline, Protease Hemmstoffe, Plasmine, Lactatdehydrogenase, Kathepsine, Cytokine, Peroxidase Enzyme, freie Kortisolradikale und Wachstumsfaktoren.

10. Vorrichtung nach einem der vorangehenden Ansprüche, **dadurch gekennzeichnet, daß** die Abtast- oder Anzeigevorrichtung ein oder mehrere Enzyme aus der folgenden Gruppe von Enzymen detektiert: Metallproteinase Matrix, Gelatinase mit einem niedrigen Molekulargewicht, latente Elastase, aktive Elastase und alkaliner Phosphatase (ALP).

## Revendications

1. Appareil comprenant :
- une entrée distribuée (3, 6) pour recueillir et mélanger des fluides de plaie provenant de plusieurs endroits d'une plaie afin de fournir un mélange de fluides de plaie ;
**caractérisé par** un dispositif de détection ou d'indication (8) en communication fluidique avec ladite entrée distribuée (3, 6) afin de détecter la présence d'un marqueur de fluide de plaie dans le mélange de fluides de plaie.

2. Appareil selon la revendication 1, dans lequel l'entrée distribuée est choisie dans le groupe comprenant plusieurs tubes capillaires espacés les uns des autres, un corps fait d'un matériau absorbant, ou une combinaison de ces derniers.

3. Appareil selon la revendication 1 ou 2, dans lequel l'entrée distribuée se présente sous forme d'une feuille ou d'une plaque ayant une surface d'au moins 10 cm².

4. Appareil selon l'une quelconque des revendications précédentes, comprenant en outre un dispositif pour exercer une succion sur l'entrée distribuée.

5. Appareil selon l'une quelconque des revendications précédentes, comprenant en outre un orifice d'alimentation en liquide pour envoyer un liquide de nettoyage vers la plaie.

6. Appareil selon l'une quelconque des revendications précédentes, dans lequel le dispositif de détection ou d'indication est visible à travers une paroi latérale de l'appareil, et subit un changement visible en présence dudit marqueur de fluide de plaie.

7. Appareil selon l'une quelconque des revendications précédentes, dans lequel le dispositif de détection ou d'indication comprend un ou plusieurs partenaires de liaison immunologiques afin de lier une ou plusieurs molécules de marqueur présentes dans le fluide de plaie.

8. Appareil selon l'une quelconque des revendications précédentes, dans lequel le dispositif de détection ou d'indication comprend un substrat chromogène ou fluorogène chimioluminescent pour une enzyme présente dans le liquide de plaie.

9. Appareil selon l'une quelconque des revendications précédentes, dans lequel le dispositif de détection ou d'indication détecte une ou plusieurs molécules choisies dans le groupe comprenant : des enzymes de proétase, des propeptides de collagène, des télopeptides de collagène et des réticulations de collagène comme de la pyrridinoline, des inhibiteurs de protéase, de la plasmine, de la lactate déshydrogénase, des cathépsines, des cytokines , des enzymes de peroxydase, des radicaux libres de cortisol, et des facteurs de croissance.

10. Appareil selon l'une quelconque des revendications précédentes, dans lequel le dispositif de détection ou d'indication détecte une ou plusieurs enzymes choisies dans le groupe comprenant des métalloprotéinases en matrice, de la gélatinase de faible poids moléculaire, des élastases latentes ou actives et de la phosphatase alcaline (ALP).
